# EUROPEAN PATENT SPECIFICATION

(11) **EP 4 504 087 B1**
(45) Date of publication and mention of the grant of the patent: **11.11.2026**
(21) Application number: 23721050.5
(22) Date of filing: 23.03.2023
(51) Int. Cl.: A61B 18/26, A61B 18/24, A61M 25/10, A61B 17/22, G02B 6/24, A61B 18/00

(54) **OPTICAL CONNECTOR ASSEMBLY FOR INTRAVASCULAR LITHOTRIPSY DEVICE**
OPTISCHE STECKERANORDNUNG FÜR INTRAVASKULÄRE LITHOTRIPSIEVORRICHTUNG
ENSEMBLE CONNECTEUR OPTIQUE POUR DISPOSITIF DE LITHOTRITIE INTRAVASCULAIRE

(30) Priority: 02.04.2022 US 202263326844 P; 22.03.2023 US 202318125050
(43) Date of publication of application: 12.02.2025
(60) Divisional application: 26210622.2
(73) Proprietor: Boston Scientific Scimed, Inc., Maple Grove, Minnesota 55311 (US)
(72) Inventor: SCHULTHEIS, Eric, San Clement, CA 92673 (US); SALINAS, Alvin, San Marcos, CA 92069 (US); DAHL, Peter, Riverside, CA 92506 (US)
(74) Representative: Vossius & Partner Patentanwälte Rechtsanwälte mbB
(86) International application number: PCT/US2023/016152
(87) International publication number: WO 2023/192109

(56) References cited:
- US-A1- 2006 098 921
- US-A1- 2021 369 348
- US-A1- 2021 386 479

## Description

### BACKGROUND

Vascular lesions within vessels in the body can be associated with an increased risk for major adverse events, such as myocardial infarction, embolism, deep vein thrombosis, stroke, and the like. Severe vascular lesions, such as severely calcified vascular lesions, can be difficult to treat and achieve patency for a physician in a clinical setting.

Vascular lesions may be treated using interventions such as drug therapy, balloon angioplasty, atherectomy, stent placement, vascular graft bypass, to name a few. Such interventions may not always be ideal or may require subsequent treatment to address the lesion.

Intravascular lithotripsy is one method that has been recently used with some success for breaking up vascular lesions within vessels in the body. Intravascular lithotripsy utilizes a combination of pressure waves and bubble dynamics that are generated intravascularly in a fluid-filled balloon catheter. In particular, during an intravascular lithotripsy treatment, a high energy source is used to generate plasma and ultimately pressure waves as well as a rapid bubble expansion within a fluid-filled balloon to crack calcification at a treatment site within the vasculature that includes one or more vascular lesions. The associated rapid bubble formation from the plasma initiation and resulting localized fluid velocity within the balloon transfers mechanical energy through the incompressible fluid to impart a fracture force on the intravascular calcium, which is opposed to the balloon wall. The rapid change in fluid momentum upon hitting the balloon wall is known as hydraulic shock, or water hammer.

There is an ongoing desire to enhance vessel patency and optimization of therapy delivery parameters within an intravascular lithotripsy catheter system in a manner that is relatively easy to control and is consistently manufacturable.

US2021/369348A1 discloses, according to its abstract, "A catheter system for treating a vascular lesion within or adjacent to a vessel wall within a body of a patient includes a single light source that generates light energy, a first light guide and a second light guide, and a multiplexer. The first light guide and the second light guide are each configured to selectively receive light energy from the light source. The multiplexer receives the light energy from the light source in the form of a source beam and selectively directs the light energy from the light source in the form of individual guide beams to each of the first light guide and the second light guide. The multiplexer includes a system of optical valves arranged in a linear sequence within the multiplexer. The system of optical valves includes an individual valve that receives the light energy from the light source".

US2006/098921A1 discloses, according to its abstract, "An improved hybrid connector for rapidly, reliably, and reversibly making mixed optical and electrical connections has a plug with one or more centrally located fiber-containing optical ferrules and one or more peripheral electrical contacts, and second, a socket with interior electrical contacts and a fiber-bearing central floating optical connector. Insertion and rotation of a shaft of the plug into a bore of the socket connects the respective electrical contacts of the shaft and socket, and nearly-simultaneously couples the respective optical fibers of the plug ferrule and socket connector, thus creating a rapid, reversible, and low- cost hybrid connection optimized for both the electrical and optical requirements of a mixed optical and electrical connection".

US2021/386479A1 discloses, according to its abstract, "A catheter system for placement within a treatment site at a vessel wall or a heart valve includes an energy source, a balloon, an energy guide , and a tissue identification system. The energy source generates energy. The balloon is positionable substantially adjacent to the treatment site. The balloon has a balloon wall that defines a balloon interior. The balloon is configured to retain a balloon fluid within the balloon interior. The energy guide is configured to receive energy from the energy source and guide the energy into the balloon interior so that plasma is formed in the balloon fluid within the balloon interior. The tissue identification system is configured to spectroscopically analyze tissue within the treatment site. A method for treating a treatment site within or adjacent to a vessel wall or a heart valve can utilize any of the catheter systems described herein".

### SUMMARY

The present invention is defined by the appended claims. A first aspect is directed toward a catheter system for placement within a blood vessel having a vessel wall. The catheter system can be used by a user for treating a treatment site within or adjacent to the vessel wall within a body of a patient. In various embodiments, the catheter system includes a system console, one or more energy guides, and an optical connector assembly. The system console includes an energy source and a console connection aperture. The one or more energy guides are configured to receive energy from the energy source. The optical connector assembly includes a guide coupling housing that retains at least a portion of each of the one or more energy guides. The guide coupling housing is configured to be mechanically connected to the system console with at least a portion of the guide coupling housing being configured to fit and be selectively retained within the console connection aperture so that the one or more energy guides are adjustably and more precisely aligned within the guide coupling housing and relative to the energy from the energy source to receive the energy from the energy source.

The optical connector assembly further includes a plurality of ferrules, and each of the plurality of ferrules is configured to retain a portion of one of the one or more energy guides.

The optical connector assembly further includes a ferrule housing having a plurality of positioning apertures that are each configured to retain at least a portion of one of the plurality of ferrules spaced apart from one another. Each of the plurality of positioning apertures is larger than a diameter of the ferrule that is retained therein to allow the ferrule to move relative to the positioning aperture.

In some embodiments, the optical connector assembly further includes a position compensator that is configured to provide a spring force to keep the ferrules in an aligned position relative to the console connection aperture while still allowing the ferrules to move relative to the ferrule housing.

In certain embodiments, the ferrule housing is adjustably positioned within the guide coupling housing so that the ferrule housing is movable relative to the guide coupling housing.

In some embodiments, the optical connector assembly further includes a resilient plate that is configured to control movement of the ferrule housing within the guide coupling housing.

In certain embodiments, the guide coupling housing includes a console facing side, and the plurality of ferrules are recessed from the console facing side of the guide coupling housing.

In some embodiments, the guide coupling housing defines a housing cavity therein, and each of the plurality of ferrules, the ferrule housing, the position compensator, and the resilient plate are retained within the housing cavity.

In certain embodiments, the guide coupling housing is formed from a first housing member and a second housing member that are selectively connected together to form the guide coupling housing and to define the housing cavity therein.

In some embodiments, the optical connector assembly further includes a sealing member that seals the connection between the guide coupling housing and the console connection aperture.

In one embodiment, the sealing member is in the form of a face gasket.

In certain embodiments, the optical connector assembly further includes a contaminant inhibitor that is positionable about at least a portion of the guide coupling housing, the contaminant inhibitor being configured to inhibit dust and particulates from contaminating a face of each of the one or more energy guides.

In one embodiment, the contaminant inhibitor is disposable.

In some embodiments, the optical connector assembly further includes a locking mechanism that is configured to selectively lock the guide coupling housing in position when the guide coupling housing is being retained within the console connection aperture.

In certain embodiments, the system console further includes an optical sensor and an actuator; and wherein the optical sensor is configured to sense a position of the guide coupling housing relative to the console connection aperture, and is further configured to initiate the actuator that mechanically draws the guide coupling housing into place within the console connection aperture.

In some embodiments, the optical connector assembly further includes a guide bundler that is configured to provide strain relief while bringing the one or more energy guides together to form an energy guide bundle.

In certain embodiments, the guide bundler includes a shaft jacket within which all of the one or more energy guides are retained as the energy guide bundle.

In one embodiment, the guide bundler further includes a locking crimp that is configured to tightly bunch the one or more energy guides together to form the energy guide bundle.

In various embodiments, the catheter system further includes a balloon that is configured to be positioned substantially adjacent to the treatment site, the balloon including a balloon wall that defines a balloon interior, the balloon being configured to retain a catheter fluid within the balloon interior.

In some embodiments, the balloon is selectively inflatable with the catheter fluid to expand to an inflated state, wherein when the balloon is in the inflated state the balloon wall is configured to be positioned substantially adjacent to the treatment site.

In certain embodiments, each of the one or more energy guides includes a guide distal end that is configured to be positioned within the balloon interior.

In some embodiments, each of the one or more energy guides is configured to guide the energy from the energy source through the energy guide and into the balloon interior.

In certain embodiments, each of the one or more energy guides guiding the energy from the energy source into the balloon interior generates a plasma bubble in the catheter fluid within the balloon interior.

In some embodiments, energy from the plasma bubble is directed toward a portion of the balloon wall that is positioned substantially adjacent to the treatment site.

In certain embodiments, each of the one or more energy guides generates one or more pressure waves in the catheter fluid that impart a force upon the treatment site.

In many embodiments, at least one of the one or more energy guides includes an optical fiber.

In various embodiments, the energy source includes a laser.

In other embodiments, the energy source is a high voltage energy source that provides pulses of high voltage.

In some embodiments, at least one of the one or more energy guides includes an electrode pair including spaced apart electrodes that extend into the balloon interior, and pulses of high voltage from the energy source are applied to the electrodes and form an electrical arc across the electrodes.

This summary is an overview of some of the teachings of the present application and is not intended to be an exclusive or exhaustive treatment of the present subject matter. Further details are found in the detailed description and appended claims. The invention is defined in the claims. Other embodiments, methods, items etc. are not a part of the invention and are disclosed for illustrative purposes only.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a simplified schematic cross-sectional view illustration of an embodiment of a catheter system in accordance with various embodiments;
Figure 2 is a simplified perspective view illustration of an embodiment of an optical connector assembly having features of the present invention that can be included as part of the catheter system of Figure 1;
Figure 3 is a simplified top view illustration of a portion of the optical connector assembly illustrated in Figure 2;
Figure 4 is a simplified end view illustration of the optical connector assembly illustrated in Figure 2; and
Figure 5 is a simplified top view illustration of another portion of the optical connector assembly illustrated in Figure 2.

### DESCRIPTION

Treatment of vascular lesions can reduce major adverse events or death in affected subjects. As referred to herein, a major adverse event is one that can occur anywhere within the body due to the presence of a vascular lesion. Major adverse events can include, but are not limited to, major adverse cardiac events, major adverse events in the peripheral or central vasculature, major adverse events in the brain, major adverse events in the musculature, or major adverse events in any of the internal organs.

As used herein, the terms "treatment site", "intravascular lesion" and "vascular lesion" can be used interchangeably unless otherwise noted. As such, the intravascular lesions and/or the vascular lesions are sometimes referred to herein simply as "lesions".

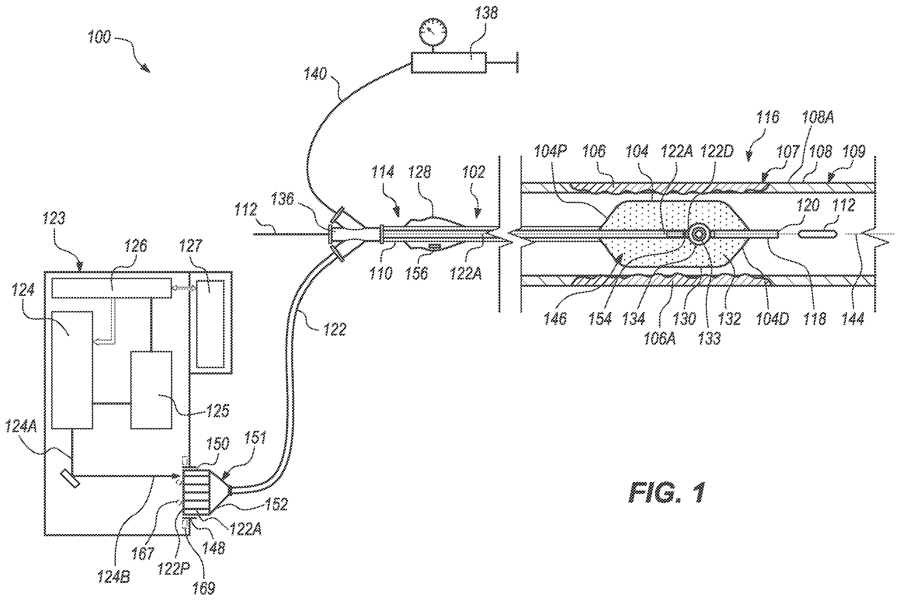

The catheter systems disclosed herein can include many different forms. Referring now to Figure 1, a simplified schematic cross-sectional view illustration is shown of a catheter system 100 in accordance with various embodiments. The catheter system 100 is suitable for imparting pressure waves to induce fractures in one or more vascular lesions within or adjacent a vessel wall of a blood vessel or on or adjacent to a heart valve within a body of a patient. In the embodiment illustrated in Figure 1, the catheter system 100 can include one or more of a catheter 102, an energy guide bundle 122 including one or more energy guides 122A, a system console 123 including one or more of an energy source 124, a power source 125, a system controller 126, and a graphic user interface 127 (a "GUI"), a handle assembly 128, and a fluid pump 138. Alternatively, the catheter system 100 can include more components or fewer components than those specifically illustrated and described in relation to Figure 1.

The catheter 102 is configured to move to the treatment site 106 within or adjacent to a vessel wall 108A of a blood vessel 108 within a body 107 of a patient 109. The treatment site 106 can include one or more vascular lesions 106A such as calcified vascular lesions, for example. Additionally, or in the alternative, the treatment site 106 can include vascular lesions 106A such as fibrous vascular lesions. Still alternatively, in some implementations, the catheter 102 can be used at a treatment site 106 within or adjacent to a heart valve within the body 107 of the patient 109.

The catheter 102 can include an inflatable balloon 104 (sometimes referred to herein simply as a "balloon"), a catheter shaft 110, and a guidewire 112. The balloon 104 can be coupled to the catheter shaft 110. The balloon 104 can include a balloon proximal end 104P and a balloon distal end 104D. The catheter shaft 110 can extend from a proximal portion 114 of the catheter system 100 to a distal portion 116 of the catheter system 100. The catheter shaft 110 can include a longitudinal axis 144. The catheter 102 and/or the catheter shaft 110 can also include a guidewire lumen 118 which is configured to move over the guidewire 112. As utilized herein, the guidewire lumen 118 defines a conduit through which the guidewire 112 extends. The catheter shaft 110 can further include an inflation lumen (not shown) and/or various other lumens for various other purposes. In some embodiments, the catheter 102 can have a distal end opening 120 and can accommodate and be tracked over the guidewire 112 as the catheter 102 is moved and positioned at or near the treatment site 106. In some embodiments, the balloon proximal end 104P can be coupled to the catheter shaft 110, and the balloon distal end 104D can be coupled to the guidewire lumen 118.

The balloon 104 includes a balloon wall 130 that defines a balloon interior 146. The balloon 104 can be selectively inflated with a catheter fluid 132 to expand from a deflated state suitable for advancing the catheter 102 through a patient's vasculature, to an inflated state (as shown in Figure 1) suitable for anchoring the catheter 102 in position relative to the treatment site 106. Stated in another manner, when the balloon 104 is in the inflated state, the balloon wall 130 of the balloon 104 is configured to be positioned substantially adjacent to the treatment site 106. It is appreciated that although Figure 1 illustrates the balloon wall 130 of the balloon 104 being shown spaced apart from the treatment site 106 of the blood vessel 108 when in the inflated state, this is done for ease of illustration. It is recognized that the balloon wall 130 of the balloon 104 will typically be substantially directly adjacent to and/or abutting the treatment site 106 when the balloon 104 is in the inflated state.

The balloon 104 suitable for use in the catheter system 100 includes those that can be passed through the vasculature of a patient 109 when in the deflated state. In some embodiments, the balloons 104 are made from silicone. In other embodiments, the balloon 104 can be made from materials such as polydimethylsiloxane (PDMS), polyurethane, polymers such as PEBAX^{™} material, nylon, or any other suitable material.

The balloon 104 can have any suitable diameter (in the inflated state). In various embodiments, the balloon 104 can have a diameter (in the inflated state) ranging from less than one millimeter (mm) up to 25 mm. In some embodiments, the balloon 104 can have a diameter (in the inflated state) ranging from at least 1.5 mm up to 14 mm. In some embodiments, the balloon 104 can have a diameter (in the inflated state) ranging from at least two mm up to five mm.

In some embodiments, the balloon 104 can have a length ranging from at least three mm to 300 mm. More particularly, in some embodiments, the balloon 104 can have a length ranging from at least eight mm to 200 mm. It is appreciated that a balloon 104 having a relatively longer length can be positioned adjacent to larger treatment sites 106, and, thus, may be usable for imparting pressure waves onto and inducing fractures in larger vascular lesions 106A or multiple vascular lesions 106A at precise locations within the treatment site 106. It is further appreciated that a longer balloon 104 can also be positioned adjacent to multiple treatment sites 106 at any one given time.

In some embodiments, the balloon 104 can include a drug eluting coating or a drug eluting stent structure. The drug eluting coating or drug eluting stent can include one or more therapeutic agents including anti-inflammatory agents, anti-neoplastic agents, anti-angiogenic agents, and the like.

The catheter fluid 132 can be a fluid, such as a liquid or a gas. Some examples of the catheter fluid 132 suitable for use can include, but are not limited to one or more of water, saline, contrast medium, fluorocarbons, perfluorocarbons, gases, such as carbon dioxide, or any other suitable catheter fluid 132.

The catheter shaft 110 of the catheter 102 can be coupled to the one or more energy guides 122A of the energy guide bundle 122 that are in optical communication with the energy source 124. The energy guide(s) 122A can be disposed along the catheter shaft 110 and within the balloon 104. In some embodiments, each energy guide 122A can be an optical fiber and the energy source 124 can be a laser. The energy source 124 can be in optical communication with the energy guides 122A at the proximal portion 114 of the catheter system 100.

In some embodiments, the catheter shaft 110 can be coupled to multiple energy guides 122A such as a first energy guide, a second energy guide, a third energy guide, etc., which can be disposed at any suitable positions about and/or relative to the guidewire lumen 118 and/or the catheter shaft 110. For example, in certain non-exclusive embodiments, two energy guides 122A can be spaced apart by approximately 180 degrees about the circumference of the guidewire lumen 118 and/or the catheter shaft 110; three energy guides 122A can be spaced apart by approximately 120 degrees about the circumference of the guidewire lumen 118 and/or the catheter shaft 110; four energy guides 122A can be spaced apart by approximately 90 degrees about the circumference of the guidewire lumen 118 and/or the catheter shaft 110; six energy guides 122A can be spaced apart by approximately 60 degrees about the circumference of the guidewire lumen 118 and/or the catheter shaft 110; eight energy guides 122A can be spaced apart by approximately 45 degrees about the circumference of the guidewire lumen 118 and/or the catheter shaft 110; or ten energy guides 122A can be spaced apart by approximately 36 degrees about the circumference of the guidewire lumen 118 and/or the catheter shaft 110. Still alternatively, multiple energy guides 122A need not be uniformly spaced apart from one another about the circumference of the guidewire lumen 118 and/or the catheter shaft 110. More particularly, it is further appreciated that the energy guides 122A can be disposed uniformly or non-uniformly about the guidewire lumen 118 and/or the catheter shaft 110 to achieve the desired effect in the desired locations.

The catheter system 100 and/or the energy guide bundle 122 can include any number of energy guides 122A in optical communication with the energy source 124 at the proximal portion 114, and with the catheter fluid 132 within the balloon interior 146 of the balloon 104 at the distal portion 116. For example, in some embodiments, the catheter system 100 and/or the energy guide bundle 122 can include from one energy guide 122A to greater than 30 energy guides 122A. Alternatively, in other embodiments, the catheter system 100 and/or the energy guide bundle 122 can include greater than 30 energy guides 122A.

The energy guides 122A can have any suitable design for purposes of generating plasma and/or pressure waves in the catheter fluid 132 within the balloon interior 146. More particularly, although the catheter systems 100 are often described with the energy source 124 as a light source and the one or more energy guides 122A as light guides, the catheter system 100 can alternatively include any suitable energy source 124 and energy guides 122A for purposes of generating the desired plasma in the catheter fluid 132 within the balloon interior 146. For example, in one non-exclusive alternative embodiment, the energy source 124 can be configured to provide high voltage pulses, and each energy guide 122A can include an electrode pair including spaced apart electrodes that extend into the balloon interior 146. In such embodiment, each pulse of high voltage is applied to the electrodes and forms an electrical arc across the electrodes, which, in turn, generates the plasma and forms the pressure waves in the catheter fluid 132 that are utilized to provide the fracture force onto the vascular lesions 106A at the treatment site 106. Still alternatively, the energy source 124 and/or the energy guides 122A can have another suitable design and/or configuration.

In certain embodiments, the energy guides 122A can include an optical fiber or flexible light pipe. The energy guides 122A can be thin and flexible and can allow light signals to be sent with very little loss of strength. The energy guides 122A can include a core surrounded by a cladding about its circumference. In some embodiments, the core can be a cylindrical core or a partially cylindrical core. The core and cladding of the energy guides 122A can be formed from one or more materials, including but not limited to one or more types of glass, silica, or one or more polymers. The energy guides 122A may also include a protective coating, such as a polymer. It is appreciated that the index of refraction of the core will be greater than the index of refraction of the cladding.

Each energy guide 122A can guide energy along its length between a guide proximal end 122P and a guide distal end 122D that is positioned within the balloon interior 146.

The energy guides 122A can assume many configurations about and/or relative to the catheter shaft 110 of the catheter 102. In some embodiments, the energy guides 122A can run parallel to the longitudinal axis 144 of the catheter shaft 110. In some embodiments, the energy guides 122A can be physically coupled to the catheter shaft 110. In other embodiments, the energy guides 122A can be disposed along a length of an outer diameter of the catheter shaft 110. In yet other embodiments, the energy guides 122A can be disposed within one or more energy guide lumens within the catheter shaft 110.

The energy guides 122A can also be disposed at any suitable positions about the circumference of the guidewire lumen 118 and/or the catheter shaft 110. The guide distal end 122D of each of the energy guides 122A can be disposed at any suitable longitudinal position relative to the length of the balloon 104 and/or relative to the length of the guidewire lumen 118 to more effectively and more precisely impart pressure waves for purposes of disrupting the vascular lesions 106A at the treatment site 106.

In certain embodiments, the energy guides 122A can include one or more photoacoustic transducers 154, where each photoacoustic transducer 154 can be in optical communication with the energy guide 122A within which it is disposed. In some embodiments, the photoacoustic transducers 154 can be in optical communication with the guide distal end 122D of the energy guide 122A. In such embodiments, the photoacoustic transducers 154 can have a shape that corresponds with and/or conforms to the guide distal end 122D of the energy guide 122A.

The photoacoustic transducer 154 is configured to convert light energy into an acoustic wave at or near the guide distal end 122D of the energy guide 122A. The direction of the acoustic wave can be tailored by changing an angle of the guide distal end 122D of the energy guide 122A.

In certain embodiments, the photoacoustic transducers 154 disposed at the guide distal end 122D of the energy guide 122A can assume the same shape as the guide distal end 122D of the energy guide 122A. For example, in certain non-exclusive embodiments, the photoacoustic transducer 154 and/or the guide distal end 122D can have a conical shape, a convex shape, a concave shape, a bulbous shape, a square shape, a stepped shape, a half-circle shape, an ovoid shape, and the like. The energy guide 122A can further include additional photoacoustic transducers 154 disposed along one or more side surfaces of the length of the energy guide 122A.

In some embodiments, the energy guides 122A can further include one or more diverting features or "diverters" (not shown in Figure 1), such as within the energy guide 122A and/or near the guide distal end 122D of the energy guide 122A, that are configured to direct energy from the energy guide 122A toward a side surface which can be located at or near the guide distal end 122D of the energy guide 122A, before the energy is directed toward the balloon wall 130. A diverting feature can include any feature of the system that diverts energy from the energy guide 122A away from its axial path toward a side surface of the energy guide 122A. The energy guides 122A can each include one or more optical windows disposed along the longitudinal or circumferential surfaces of each energy guide 122A and in optical communication with a diverting feature. Stated in another manner, the diverting features can be configured to direct energy in the energy guide 122A toward a side surface that is at or near the guide distal end 122D, where the side surface is in optical communication with an optical window. The optical windows can include a portion of the energy guide 122A that allows energy to exit the energy guide 122A from within the energy guide 122A, such as a portion of the energy guide 122A lacking a cladding material on or about the energy guide 122A.

Examples of the diverting features suitable for use include a reflecting element, a refracting element, and a fiber diffuser. The diverting features suitable for focusing energy away from the tip of the energy guides 122A can include, but are not to be limited to, those having a convex surface, a gradient-index (GRIN) lens, and a mirror focus lens. Upon contact with the diverting feature, the energy is diverted within the energy guide 122A to one or more of a plasma generator 133 and the photoacoustic transducer 154 that is in optical communication with a side surface of the energy guide 122A. When utilized, the photoacoustic transducer 154 then converts light energy into an acoustic wave that extends away from the side surface of the energy guide 122A.

As noted above, in the embodiment illustrated in Figure 1, the system console 123 can include one or more of the energy source 124, the power source 125, the system controller 126, and the GUI 127. Alternatively, the system console 123 can include greater or fewer components than those specifically illustrated in Figure 1. For example, in certain non-exclusive alternative embodiments, the system console 123 can be designed without the GUI 127. Still alternatively, one or more of the energy source 124, the power source 125, the system controller 126, and the GUI 127 can be provided at any suitable location within the catheter system 100, including outside of or remotely from the system console 123.

The system console 123 and the components included therewith can be operatively coupled to the catheter 102, the energy guide bundle 122, and/or the remainder of the catheter system 100. For example, in some embodiments, as illustrated in Figure 1, the system console 123 can include a console connection aperture 148 (also sometimes referred to generally as a "socket" or a "console receptacle") by which the energy guide bundle 122 is mechanically coupled to the system console 123. In such embodiments, the energy guide bundle 122 can include an optical connector assembly 151 having a guide coupling housing 150 (also sometimes referred to generally as a "connector housing") that houses a portion, such as the guide proximal end 122P, of each of the energy guides 122A. At least a portion of the guide coupling housing 150 is configured to fit and be selectively retained within the console connection aperture 148 to provide the mechanical coupling between the energy guide bundle 122 and the system console 123.

As described in greater detail herein, in various embodiments, the optical connector assembly 151 is configured to ensure proper alignment and coupling of the energy guide bundle 122 and/or each of the one or more energy guides 122A to the system console 123 so that energy from the energy source 124 is more precisely and accurately directed into the guide proximal end 122P of each of the one or more energy guides 122A before such energy is guided by the one or more energy guides 122A into the balloon interior 146. As further described herein below, the system console 123 can also be configured to include certain features or components, such as at least one optical sensor 167 that is usable in conjunction with at least one actuator 169, that further enable the precise alignment and coupling of the energy bundle 122 and/or each of the one or more energy guides 122A to the system console 123 and/or energy from the energy source 124 that is retained therein.

The energy guide bundle 122 and/or the optical connector assembly 151 can also include a guide bundler 152 (or "shell") that provides strain relief as it brings each of the individual energy guides 122A closer together so that the energy guides 122A and/or the energy guide bundle 122 can be in a more compact form as it extends with the catheter 102 into the blood vessel 108 during use of the catheter system 100.

The energy source 124 can be selectively and/or alternatively coupled in optical communication with each of the energy guides 122A, such as to the guide proximal end 122P of each of the energy guides 122A, in the energy guide bundle 122. In particular, the energy source 124 is configured to generate energy in the form of a source beam 124A, such as a pulsed source beam, that can be selectively and/or alternatively directed to and received by each of the properly aligned energy guides 122A in the energy guide bundle 122 as an individual guide beam 124B. Alternatively, the catheter system 100 can include more than one energy source 124. For example, in one non-exclusive alternative embodiment, the catheter system 100 can include a separate energy source 124 for each of the energy guides 122A in the energy guide bundle 122.

The energy source 124 can have any suitable design. In certain embodiments, the energy source 124 can be configured to provide sub-millisecond pulses of energy from the energy source 124 that are focused onto a small spot in order to couple it into the guide proximal end 122P of the energy guide 122A. Such pulses of energy are then directed and/or guided along the energy guides 122A to a location within the balloon interior 146 of the balloon 104, thereby inducing plasma formation in the catheter fluid 132 within the balloon interior 146 of the balloon 104, such as via the plasma generator 133 that can be located at or near the guide distal end 122D of the energy guide 122A. In particular, in such embodiments, the energy emitted at the guide distal end 122D of the energy guide 122A is directed toward and energizes the plasma generator 133 to form the plasma in the catheter fluid 132 within the balloon interior 146. The plasma formation causes rapid bubble formation, and imparts pressure waves upon the treatment site 106. An exemplary plasma-induced bubble 134 is illustrated in Figure 1.

In various non-exclusive alternative embodiments, the sub-millisecond pulses of energy from the energy source 124 can be delivered to the treatment site 106 at a frequency of between approximately one hertz (Hz) and 5000 Hz, between approximately 30 Hz and 1000 Hz, between approximately ten Hz and 100 Hz, or between approximately one Hz and 30 Hz. Alternatively, the sub-millisecond pulses of energy can be delivered to the treatment site 106 at a frequency that can be greater than 5000 Hz or less than one Hz, or any other suitable range of frequencies.

It is appreciated that although the energy source 124 is typically utilized to provide pulses of energy, the energy source 124 can still be described as providing a single source beam 124A, i.e. a single pulsed source beam.

The energy sources 124 suitable for use can include various types of light sources including lasers and lamps. Alternatively, the energy sources 124 can include any suitable type of energy source.

Suitable lasers can include short pulse lasers on the sub-millisecond timescale. In some embodiments, the energy source 124 can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths and energy levels that can be employed to achieve plasma in the catheter fluid 132 of the catheter 102. In various non-exclusive alternative embodiments, the pulse widths can include those falling within a range including from at least ten ns to 3000 ns, at least 20 ns to 100 ns, or at least one ns to 500 ns. Alternatively, any other suitable pulse width range can be used.

Exemplary nanosecond lasers can include those within the UV to IR spectrum, spanning wavelengths of about ten nanometers (nm) to one millimeter (mm). In some embodiments, the energy sources 124 suitable for use in the catheter systems 100 can include those capable of producing light at wavelengths of from at least 750 nm to 2000 nm. In other embodiments, the energy sources 124 can include those capable of producing light at wavelengths of from at least 700 nm to 3000 nm. In still other embodiments, the energy sources 124 can include those capable of producing light at wavelengths of from at least 100 nm to ten micrometers (µm). Nanosecond lasers can include those having repetition rates of up to 200 kHz.

In some embodiments, the laser can include a Q-switched thulium:yttrium-aluminum-garnet (Tm:YAG) laser. In other embodiments, the laser can include a neodymium: yttrium-aluminum-garnet (Nd:YAG) laser, holmium:yttrium-aluminum-garnet (Ho:YAG) laser, erbium:yttrium-aluminum-garnet (Er:YAG) laser, excimer laser, helium-neon laser, carbon dioxide laser, as well as doped, pulsed, fiber lasers.

In certain embodiments, the energy source 124 can include a plurality of lasers that are grouped together in series. In yet other embodiments, the energy source 124 can include one or more low energy lasers that are fed into a high energy amplifier, such as a master oscillator power amplifier (MOPA). In still yet other embodiments, the energy source 124 can include a plurality of lasers that can be combined in parallel or in series to provide the energy needed to create the plasma bubble 134 in the catheter fluid 132.

The catheter system 100 can generate pressure waves having maximum pressures in the range of at least one megapascal (MPa) to 100 MPa. The maximum pressure generated by a particular catheter system 100 will depend on the energy source 124, the absorbing material, the bubble expansion, the propagation medium, the balloon material, and other factors. In various non-exclusive alternative embodiments, the catheter systems 100 can generate pressure waves having maximum pressures in the range of at least approximately two MPa to 50 MPa, at least approximately two MPa to 30 MPa, or approximately at least 15 MPa to 25 MPa.

The pressure waves can be imparted upon the treatment site 106 from a distance within a range from at least approximately 0.1 millimeters (mm) to greater than approximately 25 mm extending radially from the energy guides 122A when the catheter 102 is placed at the treatment site 106. In various non-exclusive alternative embodiments, the pressure waves can be imparted upon the treatment site 106 from a distance within a range from at least approximately ten mm to 20 mm, at least approximately one mm to ten mm, at least approximately 1.5 mm to four mm, or at least approximately 0.1 mm to ten mm extending radially from the energy guides 122A when the catheter 102 is placed at the treatment site 106. In other embodiments, the pressure waves can be imparted upon the treatment site 106 from another suitable distance that is different than the foregoing ranges. In some embodiments, the pressure waves can be imparted upon the treatment site 106 within a range of at least approximately two MPa to 30 MPa at a distance from at least approximately 0.1 mm to ten mm. In some embodiments, the pressure waves can be imparted upon the treatment site 106 from a range of at least approximately two MPa to 25 MPa at a distance from at least approximately 0.1 mm to ten mm. Still alternatively, other suitable pressure ranges and distances can be used.

The power source 125 is electrically coupled to and is configured to provide necessary power to each of the energy source 124, the system controller 126, the GUI 127, and the handle assembly 128. The power source 125 can have any suitable design for such purposes.

The system controller 126 is electrically coupled to and receives power from the power source 125. The system controller 126 is coupled to and is configured to control operation of each of the energy source 124 and the GUI 127. The system controller 126 can include one or more processors or circuits for purposes of controlling the operation of at least the energy source 124 and the GUI 127. For example, the system controller 126 can control the energy source 124 for generating pulses of energy as desired and/or at any desired firing rate.

The system controller 126 can also be configured to control operation of other components of the catheter system 100 such as the positioning of the catheter 102 adjacent to the treatment site 106, the inflation of the balloon 104 with the catheter fluid 132, etc. Further, or in the alternative, the catheter system 100 can include one or more additional controllers that can be positioned in any suitable manner for purposes of controlling the various operations of the catheter system 100. For example, in certain embodiments, an additional controller and/or a portion of the system controller 126 can be positioned and/or incorporated within the handle assembly 128.

The GUI 127 is accessible by the user or operator of the catheter system 100. The GUI 127 is electrically connected to the system controller 126. With such design, the GUI 127 can be used by the user or operator to ensure that the catheter system 100 is effectively utilized to impart pressure onto and induce fractures into the vascular lesions 106A at the treatment site 106. The GUI 127 can provide the user or operator with information that can be used before, during and after use of the catheter system 100. In one embodiment, the GUI 127 can provide static visual data and/or information to the user or operator. In addition, or in the alternative, the GUI 127 can provide dynamic visual data and/or information to the user or operator, such as video data or any other data that changes over time during use of the catheter system 100. In various embodiments, the GUI 127 can include one or more colors, different sizes, varying brightness, etc., that may act as alerts to the user or operator. Additionally, or in the alternative, the GUI 127 can provide audio data or information to the user or operator. The specifics of the GUI 127 can vary depending upon the design requirements of the catheter system 100, or the specific needs, specifications and/or desires of the user or operator.

As shown in Figure 1, the handle assembly 128 can be positioned at or near the proximal portion 114 of the catheter system 100. In this embodiment, the handle assembly 128 is coupled to the balloon 104 and is positioned spaced apart from the balloon 104. Alternatively, the handle assembly 128 can be positioned at another suitable location.

The handle assembly 128 is attached to the catheter shaft 110 and is handled and used by the user or operator to operate, position and control the catheter 102. The design and specific features of the handle assembly 128 can vary to suit the design requirements of the catheter system 100. In the embodiment illustrated in Figure 1, the handle assembly 128 is separate from, but in electrical and/or fluid communication with one or more of the system controller 126, the energy source 124, the fluid pump 138, and the GUI 127.

In some embodiments, the handle assembly 128 can integrate and/or include at least a portion of the system controller 126 within an interior of the handle assembly 128. For example, as shown, in certain such embodiments, the handle assembly 128 can include circuitry 156, which is electrically coupled between catheter electronics and the system console 123, and which can form at least a portion of the system controller 126. In one embodiment, the circuitry 156 can include a printed circuit board having one or more integrated circuits, or any other suitable circuitry. In an alternative embodiment, the circuitry 156 can be omitted, or can be included within the system controller 126, which in various embodiments can be positioned outside of the handle assembly 128, such as within the system console 123. It is understood that the handle assembly 128 can include fewer or additional components than those specifically illustrated and described herein.

The catheter system 100 can also include the fluid pump 138 that is configured to inflate the balloon 104 with the catheter fluid 132 as needed.

As with all embodiments illustrated and described herein, various structures may be omitted from the figures for clarity and ease of understanding. Further, the figures may include certain structures that can be omitted without deviating from the intent and scope of the invention.

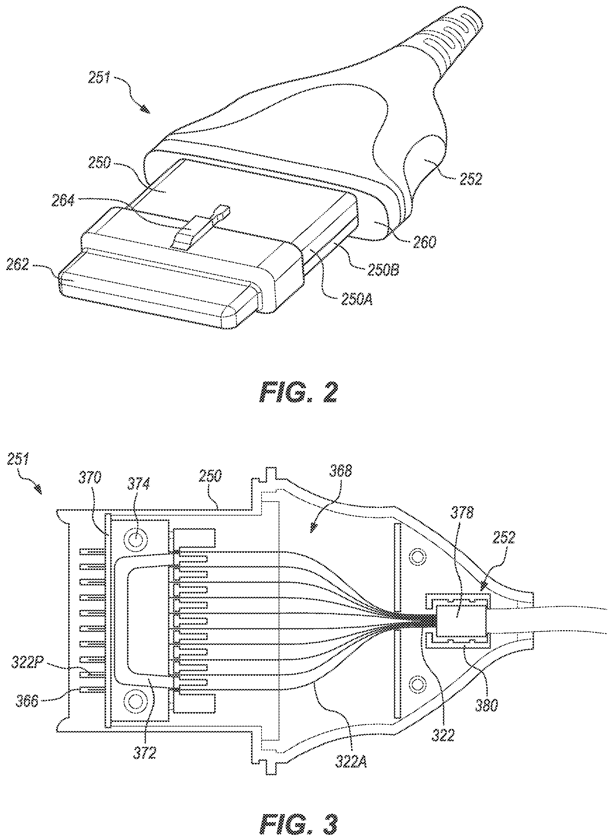

Figure 2 is a simplified perspective view illustration of an embodiment of an optical connector assembly 251 having features of the present invention that can be included as part of the catheter system 100 of Figure 1.

The design of the optical connector assembly 251 can be varied. As shown, Figure 2 illustrates various external components and features that can be included in various embodiments of the optical connector assembly 251. In particular, as illustrated, the optical connector assembly 251 can include one or more of a guide coupling (or connector) housing 250, a guide bundler 252, a sealing member 260, a contaminant inhibitor 262, and a locking mechanism 264. Alternatively, the optical connector assembly 251 can include greater or fewer external components than those specifically noted.

The purpose of the optical connector assembly 251 is to provide a means to connect ferrules 366 (illustrated, for example, in Figure 3) located within it, and thus the energy guides 122A (illustrated in Figure 1) that are positioned at least partially within the ferrules 366, into the console connection aperture 148 (or "console receptacle", illustrated in Figure 1). Thus, with the optical connector assembly 251, and the ferrules 366 and energy guides 122A retained at least partially therein, connected and aligned more precisely within the console connection aperture 148 of the system console 123 (illustrated in Figure 1), energy from the energy source 124 (illustrated in Figure 1) can be effectively and selectively coupled into each of the one or more energy guides 122A.

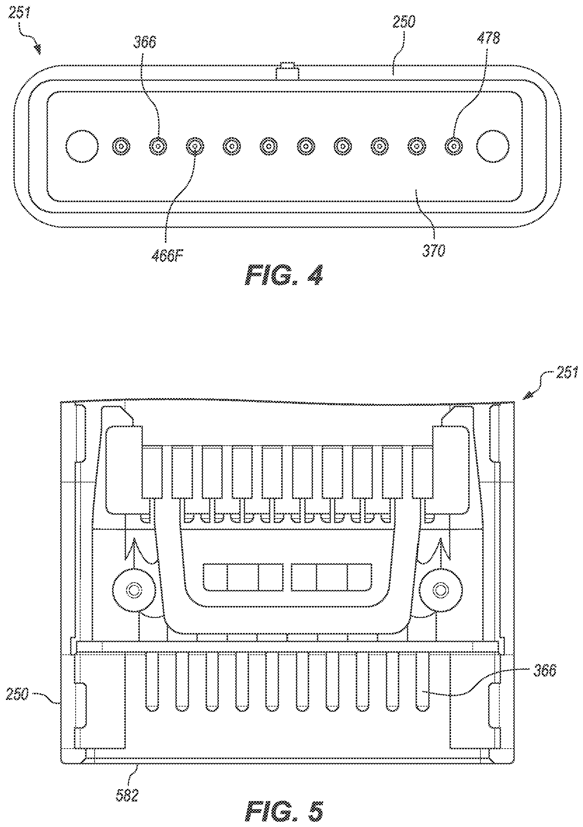

In various embodiments, the optical connector assembly 251 and/or the system console 123 (illustrated in Figure 1) can include certain features or components to better ensure the more precisely aligned connection therebetween. For example, in some embodiments, the optical connector assembly 251 can include (i) the ferrules 366 that are allowed to float relative to and/or within a ferrule housing 370 (illustrated in Figure 3) by the ferrule housing 370 having positioning apertures 476 (illustrated in Figure 4) within which at least a portion of the ferrules 366 is retained that are slightly larger than the diameter of the ferrules 366; (ii) the ferrule housing 370 that is allowed to float relative to the guide coupling housing 250 by selectively moving the ferrule housing 370 (up-and-down and/or side-to-side) relative to the guide coupling housing 250 as necessary; (iii) a position compensator 374 (illustrated in Figure 3) that is configured to provide a spring force to keep the ferrules 366 in an aligned position, while still allowing play within the ferrule housing 370; and (iv) a resilient plate 372 that is configured to control the floating of the ferrule housing 370 within the guide coupling housing 250. In certain embodiments, the system console 123 can include one or more of the optical sensors 167 (illustrated in Figure 1) that are configured to sense a position of the optical connector assembly 251 and/or the guide coupling housing 250 relative to the system console 123 and/or the console connection aperture 148, and initiate the actuator 169 (illustrated in Figure 1) that mechanically draws the optical connector assembly 251 more accurately into place within the console connection aperture 148.

During use of the catheter system 100, it is also desired to limit the amount of dust, fluids and/or other particulates that may otherwise contaminate a guide face of the guide proximal end 122P (illustrated in Figure 1) of each of the one or more energy guides 122A.

The guide coupling housing 250 is configured to house a portion of each of the energy guides 122A, such as the guide proximal end 122P, and to fit and be selectively retained within the console connection aperture 148 to provide the mechanical coupling between the energy guide bundle 122 (illustrated in Figure 1) and the system console 123. The design of the guide coupling housing 250 can be varied to suit the requirements of the catheter system 100 and/or the optical connector assembly 251. In certain embodiments, the guide coupling housing 250 can be formed from multiple housing members, such as a first housing member 250A and a second housing member 250B, that can be selectively coupled together to retain various internal components of the optical connector assembly 251 effectively within a housing cavity 368 (illustrated in Figure 3) defined therein. In one embodiment, each of the first housing member 250A and the second housing member 250B can form one-half of the guide coupling housing 250 (such as a top half and a corresponding bottom half in one non-exclusive embodiment), with each half being substantially similar to the other half. It is appreciated that the housing members 250A, 250B can be selectively coupled together in any suitable manner. Alternatively, the guide coupling housing 250 can have another suitable design.

It is appreciated that the guide coupling housing 250 and/or the individual housing members 250A, 250B can be formed from any suitable materials that provide an effective housing to protect the various components retained therein. Various internal components of the optical connector assembly 251 are illustrated and described herein below in relation to Figure 3.

The guide bundler 252 is configured to provide strain relief as it brings each of the individual energy guides 122A closer together so that the energy guides 122A and/or the energy guide bundle 122 can be in a more compact form as it extends with the catheter 102 (illustrated in Figure 1) into the blood vessel 108 (illustrated in Figure 1) during use of the catheter system 100. Certain internal components that can be included within the guide bundler 252 for purposes of providing strain relief as it brings the energy guides 122A closer together within the energy guide bundle 122 are illustrated and described herein below in relation to Figure 3.

The sealing member 260 is configured to seal the connection between the optical connector assembly 251 and the system console 123 when the guide coupling housing 250 is inserted and selectively retained within the console connection aperture 148. With such design, the sealing member 260, which can be provided in the form of a face gasket in one non-exclusive embodiment, can help to limit the amount of dust and other particulates that may otherwise be introduced into the guide coupling housing 250 and/or the guide coupler 252. In some embodiments, the sealing member 260 can be formed from a resilient material that can effectively provide a sealed connection between the guide coupling housing 250 and the console connection aperture 148. Alternatively, the sealing member 260 can be formed from another suitable material.

The contaminant inhibitor 262 is configured to limit the amount of dust, fluids and/or other particulates (also sometimes individually or collectively referred to herein as "contaminates") that may act as an impediment or otherwise contaminate the guide face of each of the one or more energy guides 122A. More particularly, as shown, the contaminant inhibitor 262 can be configured to be positioned about a portion of the guide coupling housing 250 within which the ferrules 366, and thus the guide proximal end 122P of each of the energy guides 122A, are retained. The contaminant inhibitor 262 can have any suitable design which is configured to inhibit the introduction of dust and other particulates into the guide coupling housing 250 while still permitting energy from the energy source 124 to be coupled into the guide proximal end 122P of each of the one or more energy guides 122A.

In certain embodiments, the contaminant inhibitor 262 can be disposable such that when the contaminate inhibitor 262 gets sufficiently contaminated with contaminates, the contaminant inhibitor 262 can be simply thrown away. In other embodiments, the contaminant inhibitor 262 can be reusable, such that it can be selectively removed from the guide coupling housing 250 and cleaned, and then again selectively coupled to the guide coupling housing 250 for additional use.

The locking mechanism 264 is configured to selectively lock the optical connector assembly 251 in position when it is coupled into the console connection aperture 148. More specifically, during use of the catheter system 100, as the optical connector assembly 251 is inserted into the console connection aperture 148 of the system console 123, the optical sensors 167 register it and initiate the actuator 169 that mechanically draws the optical connector assembly 251 into place and locks it in position. The locking mechanism 264 provides an effective means to thus lock the optical connector assembly 251 in such position where the optical connector assembly 251 has been inserted into the console connection aperture 148 so that it can be selectively retained therein.

Figure 3 is a simplified top view illustration of a portion of the optical connector assembly 251 (illustrated in Figure 2). More specifically, Figure 3 illustrates various internal components and features that can be included in various embodiments of the optical connector assembly 251. As shown in Figure 3, in various embodiments, the optical connector assembly 251 can internally include within the guide coupling housing 250 one or more of a plurality of ferrules 366, a ferrule housing 370, a portion of the one or more energy guides 322A, a resilient plate 372, at least one position compensator 374 (such as a silicone gasket in one non-exclusive embodiment), and at least a portion of the guide bundler 252. It is appreciated that only one of the housing members 250A, 250B of the guide coupling housing 250 is visible in Figure 3 so that the other noted components can be clearly seen positioned within the housing cavity 368 that is defined within the guide coupling housing 250.

As utilized herein, a "ferrule" is a component in fiber optics used for protecting and aligning a stripped end of the energy guide 322A (or optical fiber). During use, the energy guide 322A is inserted into the thin structure of the ferrule 366 and can be provided with an adhesive (not shown) to prevent contamination as well as to give it long-term mechanical strength. The ferrules 366 can be formed from any suitable materials for purposes of providing the desired contamination protection for the stripped guide proximal end 322P of the energy guides 322A as well as the enhanced, long-term mechanical strength.

The optical connector assembly 251 can include any suitable number of ferrules 366 within the housing cavity 368 as defined by the guide coupling housing 250, depending on the number of energy guides 322A that are to be optically connected to the energy source 124 (illustrated in Figure 1). For example, in one non-exclusive embodiment, as shown in Figure 3, the optical connector assembly 251 can include ten ferrules 366 that are each configured to retain and protect a portion, such as the guide proximal end 322P, of one of the one or more energy guides 322A. Alternatively, the optical connector assembly 251 can include greater than ten or less than ten ferrules 366.

The ferrule housing 370 is configured to provide a housing for the ferrules 366 so that the ferrules 366 can be moved and positioned collectively relative to the energy from the energy source 124, with the ferrules 366 maintained spaced apart a desired distance from one another, and so that the guide proximal end 322P of each of the energy guides 322A can be properly aligned to accurately receive energy from the energy source 124. At the side of the guide coupling housing 250 that faces the console connection aperture 148 (illustrated in Figure 1) of the system console 123 (illustrated in Figure 1), i.e. the left side in Figure 3, faces of the ferrules 366 are exposed. In certain embodiments, the ferrules 366 are allowed to float significantly in the ferrule housing 370 to allow for the ferrules 366, and thus the guide proximal end 322P of the energy guides 322A, to more accurately line up with the console connection aperture 148 of the system console 123.

Figure 4 is a simplified end view illustration of the optical connector assembly 251 illustrated in Figure 2. More particularly, Figure 4 illustrates a face 466F of each of the ferrules 366 as the ferrules 366 are retained in generally spaced apart desired positions within the ferrule housing 370. In some embodiments, as illustrated, the ferrules 366 are positioned within positioning apertures 476 that are formed into the ferrule housing 370. As shown, the positioning apertures 476 can be sized to have tolerances that enable a loose fit of the ferrules 366 within the positioning apertures 476. Stated in another manner, in certain embodiments, the positioning apertures 476 are slightly larger than a diameter of the ferrules 366 to allow the ferrule 366 to move relative to the ferrule housing 370. With such design, as noted, the ferrules 366 are allowed to float significantly in the ferrule housing 370 to allow for the ferrules 366, and thus the guide proximal end 322P (illustrated in Figure 3) of the energy guides 322A (illustrated in Figure 3), to more accurately line up with the console connection aperture 148 (illustrated in Figure 1) of the system console 123 (illustrated in Figure 1).

As the optical connector assembly 251 is advanced into the console connection aperture 148, the ferrules 366 find their place in the console connection aperture 148 due to a chamfer lead-in on the console connection aperture 148. This allows for a tight tolerance on the console connection aperture 148 and the tight tolerances of the ferrules' outer diameter to drive the fit.

In various embodiments, the ferrule housing 370 can also be selectively adjustable in position within the guide coupling housing 250 to better enable the desired alignment between the energy guides 322A and the energy from the energy source 124 (illustrated in Figure 1). Stated in another manner, in addition to the loose fit between the ferrules 366 and the positioning apertures 476 in the ferrule housing 370, the ferrule housing 370 is also allowed to float (up-and-down and/or side-to-side) inside the assembled guide coupling housing 250. With such design, enabling of the accurate and precise positioning of the guide proximal end 322P of each of the energy guides 322A relative to the energy from the energy source 124 is further enhanced.

Returning again to Figure 3, the resilient plate 372, such as a spring plate in certain embodiments, is configured to control the floating of the ferrule housing 370 within the guide coupling housing 250. More particularly, as the ferrule housing 370 is allowed to float within the guide coupling housing 250, it is desired that the ferrule housing 370 does not just float loosely without control within the guide coupling housing 250. The resilient plate 372 provides a biasing force that allows the ferrule housing 370 to float within the guide coupling housing 250 while enabling the ferrule housing 370 to be resiliently maintained in position within the guide coupling housing 250 once a desired positioning is accurately determined.

The at least one position compensator 374, such as silicone gaskets in certain non-exclusive embodiments, is configured to provide a spring force to keep the ferrules 366 in an aligned position, while still allowing play within the ferrule housing 370. However, if the ferrule housing 370 needs to adjust to accommodate fit with the ferrules 366 relative to the console connection aperture 148 and/or the energy from the energy source 124, then the ferrule housing 370 can be moved to accommodate such adjusted position. Without the at least one position compensator 374, the ferrules 366 could seize with the console connection aperture 148, due to fit interference.

The optical connector assembly 251 can include any suitable number of position compensators 374. For example, in one non-exclusive embodiment, the optical connector assembly 251 can include four position compensators 374. Alternatively, in other embodiments, the optical connector assembly 251 can include greater than four or less than four position compensators 374.

As noted above, the guide bundler 252 is configured to provide strain relief as it brings each of the individual energy guides 322A closer together so that the energy guides 322A and/or the energy guide bundle 322 can be in a more compact form as it extends with the catheter 102 (illustrated in Figure 1) into the blood vessel 108 (illustrated in Figure 1) during use of the catheter system 100 (illustrated in Figure 1). The design of the guide bundler 252 can be varied. For example, as shown in Figure 3, in certain embodiments, the guide bundler 252 can include a shaft jacket 378 within which all of the energy guides 322A are maintained as the energy guide bundle 322 extends with the catheter 102 toward the balloon 104 (illustrated in Figure 1). The guide bundler 252 can also include a locking crimp 380 that is configured to tightly bunch the energy guides together in a controlled manner to form the energy guide bundle 322.

As shown, Figure 3 also illustrates the routing of the energy guides 322A as they extend through the guide coupling housing 250 of the optical connector assembly 251. More specifically, the guide proximal end 322P of each of the energy guides 322A is positioned within one of the ferrules 366 near the side of the guide coupling housing 250 that faces the console connection aperture 148 (illustrated in Figure 1) of the system console 123, with the energy guides 322A being positioned at a desired spacing relative to one another. The energy guides 322A then extend through the guide coupling housing 250 to where they are brought closer together, or bundled together, at the guide bundler 252. The energy guide bundle 322, with the energy guides 322A positioned within the shaft jacket 378, then extends with the catheter 102 toward the balloon 104.

Figure 5 is a simplified top view illustration of another portion of the optical connector assembly 251 illustrated in Figure 2. As shown in Figure 5, in some embodiments, the ferrules 366 are positioned in a manner within the guide coupling housing 250 such that the ferrules 366 are recessed relative to a console facing side 582 of the guide coupling housing 250. With the ferrules 366 being recessed from the console facing side 582 of the guide coupling housing 250, the optical connector assembly 251 is configured to help ensure that fingers or other objects do not come into contact with faces of the energy guides 322A (illustrated in Figure 3), at the guide proximal end 322P (illustrated in Figure 3) of the energy guides 322A, which could otherwise lead to undesired contamination.

The ferrules 366 can be recessed any desired distance from the console facing side 582 of the guide coupling housing 250 depending of the specific design requirements of the optical connector assembly 251 and/or the catheter system 100 (illustrated in Figure 1). In various embodiments, the catheter systems can include a catheter configured to advance to a vascular lesion, such as a calcified vascular lesion or a fibrous vascular lesion, at a treatment site located within or adjacent a blood vessel within a body of a patient. The catheter includes a catheter shaft, and an inflatable balloon that is coupled and/or secured to the catheter shaft. The balloon can include a balloon wall that defines a balloon interior. The balloon can be configured to receive a catheter fluid within the balloon interior to expand from a deflated state suitable for advancing the catheter through a patient's vasculature, to an inflated state suitable for anchoring the catheter in position relative to the treatment site.

It should be noted that, as used in this specification and the appended claims, the singular forms "a," "an," and "the" include plural referents unless the content and/or context clearly dictates otherwise. It should also be noted that the term "or" is generally employed in its sense including "and/or" unless the content or context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

It is recognized that the figures shown and described are not necessarily drawn to scale, and that they are provided for ease of reference and understanding, and for relative positioning of the structures.

## Claims

1. A catheter system for use by a user in treating a treatment site within or adjacent to a blood vessel within a body of a patient, the catheter system comprising:
a system console (123) including an energy source (124) and a console connection aperture (148), the energy source generating energy;
one or more energy guides (122A) that are configured to receive the energy from the energy source; and
an optical connector assembly including (i) a guide coupling housing (150) that retains at least a portion of each of the one or more energy guides, the guide coupling housing being configured to be mechanically connected to the system console, at least a portion of the guide coupling housing being configured to fit and be selectively retained within the console connection aperture so that the one or more energy guides are adjustably aligned within the guide coupling housing to better receive the energy from the energy source; (ii) a plurality of ferrules (366) each ferrule being configured to retain a portion of one of the one or more energy guides; and (iii) a ferrule housing (370), the ferrule housing having a plurality of positioning apertures, each positioning aperture being configured to retain at least a portion of one of the plurality of ferrules, each of the plurality of positioning apertures having a diameter that is larger than a diameter of the ferrule that is retained therein so that the ferrule is movable within the positioning aperture.

2. The catheter system of claim 1 wherein the optical connector assembly further includes a position compensator that is configured to provide a spring force to maintain the ferrules in an aligned position relative to the console connection aperture while allowing the ferrules to move relative to the ferrule housing.

3. The catheter system of claim 2 wherein the optical connector assembly further includes a resilient plate that is configured to control movement of the ferrule housing within the guide coupling housing.

4. The catheter system of claim 3 wherein the guide coupling housing defines a housing cavity, each of the plurality of ferrules, the ferrule housing, the position compensator, and the resilient plate being retained within the housing cavity.

5. The catheter system of any one of claims 1 to 4 wherein the ferrule housing is adjustably positioned within the guide coupling housing so that the ferrule housing is movable within the guide coupling housing.

6. The catheter system of any one of claims 1 to 5 wherein the guide coupling housing includes a console facing side, the plurality of ferrules being recessed from the console facing side of the guide coupling housing.

7. The catheter system of any one of claims 1 to 6 wherein the optical connector assembly further includes a sealing member that seals the connection between the guide coupling housing and the console connection aperture.

8. The catheter system of any one of claims 1 to 7 wherein the optical connector assembly further includes a contaminant inhibitor that is positionable about at least a portion of the guide coupling housing, the contaminant inhibitor being configured to inhibit contaminants from contaminating a face of at least one of the energy guides.

9. The catheter system of any one of claims 1 to 8 wherein the optical connector assembly further includes a locking mechanism that is configured to lock the guide coupling housing into position when the guide coupling housing is being retained within the console connection aperture.

10. The catheter system of any one of claims 1 to 9 wherein the system console further includes an optical sensor and an actuator, the optical sensor being configured to (i) sense a position of the guide coupling housing relative to the console connection aperture, and (ii) initiate the actuator that mechanically draws the guide coupling housing into place within the console connection aperture.

11. The catheter system of any one of claims 1 to 10 wherein the optical connector assembly further includes a guide bundler that is configured to provide strain relief while bringing the one or more energy guides together to form an energy guide bundle.

12. The catheter system of claim 11 wherein the guide bundler includes a shaft jacket within which all of the one or more energy guides are retained as the energy guide bundle.

13. The catheter system of claim 12 wherein the guide bundler further includes a locking crimp that is configured to tightly bunch the one or more energy guides together to form the energy guide bundle.

14. The catheter system of any one of claims 1 to 13 further comprising a balloon that is configured to be positioned substantially adjacent to the treatment site, the balloon including a balloon wall that defines a balloon interior, the balloon being configured to retain a catheter fluid within the balloon interior, each of the one or more energy guides including a guide distal end that is configured to be positioned within the balloon interior, each of the one or more energy guides being configured to guide the energy from the energy source through the energy guide and into the balloon interior to generate a plasma bubble in the catheter fluid within the balloon interior so that a pressure wave is generated that imparts a force upon the treatment site.

15. The catheter system of any one of claims 1 to 14 wherein at least one of the one or more energy guides includes an optical fiber, and the energy source includes a laser.

## Patentansprüche

1. Katheter-System zur Verwendung durch einen Benutzer beim Behandeln einer Behandlungsstelle in einem oder angrenzend an ein Blutgefäß in einem Körper eines Patienten, wobei das Katheter-System aufweist:
eine Systemkonsole (123), die eine Energiequelle (124) und eine Konsolenverbindungsöffnung (148) aufweist, wobei die Energiequelle Energie erzeugt;
einen oder mehrere Energieleiter (122A), die konfiguriert sind, die Energie aus der Energiequelle zu empfangen; und
eine optische Steckeranordnung mit (i) einem Leiterkopplungsgehäuse (150), das mindestens einen Abschnitt jedes des einen oder der mehreren Energieleiter hält, wobei das Leiterkopplungsgehäuse konfiguriert ist, mechanisch mit der Systemkonsole verbunden zu werden, wobei mindestens ein Teil des Leiterkopplungsgehäuses konfiguriert ist, in die Konsolenverbindungsöffnung zu passen und selektiv darin gehalten zu werden, derart, dass der eine oder die mehreren Energieleiter anpassbar in dem Leiterkopplungsgehäuse ausgerichtet sind, um die Energie aus der Energiequelle besser zu empfangen; (ii) mehreren Klemmen (366), wobei jede Klemme konfiguriert ist, einen Abschnitt eines des einen oder der mehrerer Energieleiter zu halten; und (iii) einem Klemmengehäuse (370), wobei das Klemmengehäuse mehrere Positionierungsöffnungen hat, wobei jede Positionierungsöffnung konfiguriert ist, mindestens einen Abschnitt einer der mehreren Klemmen zu halten, wobei jede der mehreren Positionierungsöffnungen einen Durchmesser hat, der größer als ein Durchmesser der darin gehaltenen Klemme ist, so dass die Klemme in der Positionierungsöffnung bewegbar ist.

2. Katheter-System nach Anspruch 1, wobei die optische Steckeranordnung ferner einen Positionskompensator aufweist, der konfiguriert ist, eine Federkraft bereitzustellen, um die Klemmen in einer ausgerichteten Position relativ zu der Konsolenverbindungsöffnung zu halten und zugleich den Klemmen zu erlauben, sich relativ zu dem Klemmengehäuse zu bewegen.

3. Katheter-System nach Anspruch 2, wobei die optische Steckeranordnung ferner eine elastische Platte aufweist, die konfiguriert ist, eine Bewegung des Klemmengehäuses in dem Leiterkopplungsgehäuse zu steuern.

4. Katheter-System nach Anspruch 3, wobei das Leiterkopplungsgehäuse einen Gehäusehohlraum definiert, wobei jede der mehreren Klemmen, das Klemmengehäuse, der Positionskompensator und die elastische Platte in dem Gehäusehohlraum gehalten werden.

5. Katheter-System nach einem der Ansprüche 1 bis 4, wobei das Klemmengehäuse in dem Leiterkopplungsgehäuse anpassbar positioniert ist, so dass das Klemmengehäuse in dem Leiterkopplungsgehäuse bewegbar ist.

6. Katheter-System nach einem der Ansprüche 1 bis 5, wobei das Leiterkopplungsgehäuse eine konsolenzugewandte Seite aufweist, wobei die mehreren Klemmen aus der konsolenzugewandten Seite des Leiterkopplungsgehäuses ausgespart sind.

7. Katheter-System nach einem der Ansprüche 1 bis 6, wobei die optische Steckeranordnung ferner ein Dichtungselement aufweist, das die Verbindung zwischen dem Leiterkopplungsgehäuse und der Konsolenverbindungsöffnung abdichtet.

8. Katheter-System nach einem der Ansprüche 1 bis 7, wobei die optische Steckeranordnung ferner einen Kontaminanten-Hemmer aufweist, der um mindestens einen Teil des Leiterkopplungsgehäuses herum positionierbar ist, wobei der Kontaminanten-Hemmer konfiguriert ist, Kontaminanten daran zu hindern, eine Fläche mindestens eines der Energieleiter zu kontaminieren.

9. Katheter-System nach einem der Ansprüche 1 bis 8, wobei die optische Steckeranordnung ferner einen Verriegelungsmechanismus aufweist, der konfiguriert ist, das Leiterkopplungsgehäuse in Position zu verriegeln, wenn das Leiterkopplungsgehäuse in der Konsolenverbindungsöffnung gehalten wird.

10. Katheter-System nach einem der Ansprüche 1 bis 9, wobei die Systemkonsole ferner einen optischen Sensor und einen Aktuator aufweist, wobei der optische Sensor konfiguriert ist, (i) eine Position des Leiterkopplungsgehäuses relativ zu der Konsolenverbindungsöffnung zu erkennen, und (ii) den Aktuator zu initiieren, der das Leiterkopplungsgehäuse in der Konsolenverbindungsöffnung mechanisch in Position zieht.

11. Katheter-System nach einem der Ansprüche 1 bis 10, wobei die optische Steckeranordnung ferner einen Leiterbündelbilder aufweist, der konfiguriert ist, Zugentlastung bereitzustellen und den einen oder die mehreren Energieleiter zusammenzubringen, um ein Energieleiterbündel zu bilden.

12. Katheter-System nach Anspruch 11, wobei der Leiterbündelbilder eine Schafthülle aufweist, in der alle des einen oder der mehreren Energieleiter als das Energieleiterbündel gehalten werden.

13. Katheter-System nach Anspruch 12, wobei der Leiterbündelbilder ferner eine Verriegelungshülse aufweist, die konfiguriert ist, den einen oder die mehreren Energieleiter fest zusammenzufassen, um das Energieleiterbündel zu bilden.

14. Katheter-System nach einem der Ansprüche 1 bis 13, ferner aufweisend einen Ballon, der konfiguriert ist, im Wesentlichen angrenzend an die Behandlungsstelle positioniert zu werden, wobei der Ballon eine Ballonwand aufweist, die einen Balloninnenraum definiert, wobei der Ballon konfiguriert ist, ein Katheter-Fluid in dem Balloninnenraum zu halten, wobei jeder des einen oder der mehreren Energieleiter ein distales Leiterende aufweist, das konfiguriert ist, in dem Balloninnenraum positioniert zu werden, wobei jeder des einen oder der mehreren Energieleiter konfiguriert ist, die Energie aus der Energiequelle durch den Energieleiter hindurch und in den Balloninnenraum zu leiten, um in dem Balloninnenraum eine Plasmablase in dem Katheter-Fluid zu erzeugen, so dass eine Druckwelle erzeugt wird, die eine Kraft auf die Behandlungsstelle ausübt.

15. Katheter-System nach einem der Ansprüche 1 bis 14, wobei mindestens einer des einen oder der mehreren Energieleiter eine optische Faser aufweist und die Energiequelle einen Laser aufweist.

## Revendications

1. Système de cathéter destiné à être utilisé par un utilisateur pour traiter un site de traitement situé dans ou de manière adjacente à un vaisseau sanguin à l'intérieur du corps d'un patient, le système de cathéter comprenant :
une console de système (123) comprenant une source d'énergie (124) et une ouverture de raccordement de console (148), la source d'énergie générant de l'énergie ;
un ou plusieurs guides d'énergie (122A) qui sont configurés pour recevoir l'énergie de la source d'énergie ; et
un ensemble connecteur optique comprenant (i) un boîtier de couplage de guide (150) qui retient au moins une partie de chacun des un ou plusieurs guides d'énergie, le boîtier de couplage de guidage étant configuré pour être mécaniquement raccordé à la console de système, au moins une partie du boîtier de couplage de guide étant configurée pour s'ajuster et être sélectivement retenue à l'intérieur de l'ouverture de raccordement de console de sorte que les un ou plusieurs guides d'énergie sont alignés, par ajustement, à l'intérieur du boîtier de couplage de guide pour mieux recevoir l'énergie provenant de la source d'énergie ; (ii) une pluralité de férules (366), chaque férule étant configurée pour retenir une partie de l'un des un ou plusieurs guides d'énergie ; et (iii) un boîtier de férule (370),
le boîtier de férule ayant une pluralité d'ouvertures de positionnement, chaque ouverture de positionnement étant configurée pour retenir au moins une partie de l'une de la pluralité de férules, chacune de la pluralité d'ouvertures de positionnement ayant un diamètre qui est plus grand qu'un diamètre de la férule qui est retenue dans cette dernière, de sorte que la férule peut se déplacer dans l'ouverture de positionnement.

2. Système de cathéter selon la revendication 1, dans lequel l'ensemble connecteur optique comprend en outre un compensateur de position qui est configuré pour fournir une force de rappel afin de maintenir les férules dans une position alignée par rapport à l'ouverture de raccordement de console tout en permettant aux férules de se déplacer par rapport au boîtier de férule.

3. Système de cathéter selon la revendication 2, dans lequel l'ensemble connecteur optique comprend en outre une plaque résiliente qui est configurée pour commander le déplacement du boîtier de férule à l'intérieur du boîtier de couplage de guide.

4. Système de cathéter selon la revendication 3, dans lequel le boîtier de couplage de guide définit une cavité de boîtier, chacun parmi la pluralité de férules, le boîtier de férule, le compensateur de position et la plaque résiliente étant retenu à l'intérieur de la cavité de boîtier.

5. Système de cathéter selon l'une quelconque des revendications 1 à 4, dans lequel le boîtier de férule est positionné, de manière réglable, à l'intérieur du boîtier de couplage de guide de sorte que le boîtier de férule est mobile à l'intérieur du boîtier de couplage de guide.

6. Système de cathéter selon l'une quelconque des revendications 1 à 5, dans lequel le boîtier de couplage de guide comprend un côté orienté vers la console, la pluralité de férules étant enfoncée par rapport au côté orienté vers la console du boîtier de couplage de guide.

7. Système de cathéter selon l'une quelconque des revendications 1 à 6, dans lequel l'ensemble connecteur optique comprend en outre un élément d'étanchéité qui scelle le raccordement entre le boîtier de couplage de guide et l'ouverture de raccordement de console.

8. Système de cathéter selon l'une quelconque des revendications 1 à 7, dans lequel l'ensemble connecteur optique comprend en outre in inhibiteur de contaminant qui peut être positionné autour d'au moins une partie du boîtier de couplage de guide, l'inhibiteur de contaminant étant configuré pour empêcher les contaminants de contaminer une face d'au moins l'un des guides d'énergie.

9. Système de cathéter selon l'une quelconque des revendications 1 à 8, dans lequel l'ensemble connecteur optique comprend en outre un mécanisme de verrouillage qui est configuré pour verrouiller le boîtier de couplage de guide en position lorsque le boîtier de couplage de guide est retenu à l'intérieur de l'ouverture de raccordement de console.

10. Système de cathéter selon l'une quelconque des revendications 1 à 9, dans lequel la console de système comprend en outre un capteur optique et un actionneur, le capteur optique étant configuré pour (i) détecter une position du boîtier de couplage de guide par rapport à l'ouverture de raccordement de console, et (ii) déclencher l'actionneur qui tire mécaniquement le boîtier de couplage de guide en place à l'intérieur de l'ouverture de raccordement de console.

11. Système de cathéter selon l'une quelconque des revendications 1 à 10, dans lequel l'ensemble connecteur optique comprend en outre un dispositif de regroupement de guides qui est configuré pour assurer une protection contre les contraintes tout en rapprochant les un ou plusieurs guides d'énergie pour former un faisceau de guides d'énergie.

12. Système de cathéter selon la revendication 11, dans lequel le dispositif de regroupement de guides comprend une chemise de tige à l'intérieur de laquelle la totalité des un ou plusieurs guides d'énergie est retenue sous la forme d'un faisceau de guides d'énergie.

13. Système de cathéter selon la revendication 12, dans lequel le dispositif de regroupement de guides comprend en outre un sertissage de verrouillage qui est configuré pour regrouper étroitement les un ou plusieurs guides d'énergie afin de former le faisceau de guides d'énergie.

14. Système de cathéter selon l'une quelconque des revendications 1 à 13, comprenant en outre un ballonnet qui est configuré pour être positionné de manière sensiblement adjacente au site de traitement, le ballonnet comprenant une paroi de ballonnet qui définit un intérieur de ballonnet, le ballonnet étant configuré pour retenir un fluide de cathéter à l'intérieur du ballonnet, chacun parmi les un ou plusieurs guides d'énergie comprenant une extrémité distale de guide qui est configurée pour être positionnée à l'intérieur du ballonnet, chacun parmi les un ou plusieurs guides d'énergie étant configuré pour guider l'énergie de la source d'énergie en passant par le guide d'énergie et dans l'intérieur du ballonnet afin de générer une bulle de plasma dans le fluide de cathéter à l'intérieur du ballonnet de sorte que l'on génère une onde de pression qui transmet une force sur le site de traitement.

15. Système de cathéter selon l'une quelconque des revendications 1 à 14, dans lequel au moins l'un des un ou plusieurs guides d'énergie comprend une fibre optique, et la source d'énergie comprend un laser.
